(19) Europäisches Patentamt
European Patent Office
Office européen des brevets

(11) Veröffentlichungsnummer: **0 014 900**
**B1**

(12) # EUROPÄISCHE PATENTSCHRIFT

(45) Veröffentlichungstag der Patentschrift: **02.06.82**

(21) Anmeldenummer: **80100600.8**

(22) Anmeldetag: **06.02.80**

(51) Int. Cl.³: **C 07 C 79/46,** C 07 C 76/02, C 07 C 103/34, A 01 N 39/04, C 07 D 211/16, C 07 D 223/04, A 01 N 43/34

(54) **Phenoxycarbonsäure-carbonylalkylester, Verfahren zu ihrer Herstellung und ihre Verwendung als Herbizide sowie als Defoliants und Desiccants.**

(30) Priorität: **17.02.79 DE 2906087**

(43) Veröffentlichungstag der Anmeldung: **03.09.80 Patentblatt 80/18**

(45) Bekanntmachung des Hinweises auf die Patenterteilung: **02.06.82 Patentblatt 82/22**

(84) Benannte Vertragsstaaten: **AT BE CH DE FR GB IT NL SE**

(56) Entgegenhaltungen: **US-A-4 093 446**

(73) Patentinhaber: **BAYER AG, Zentralbereich Patente, Marken und Lizenzen, D-5090 Leverkusen 1, Bayerwerk (DE)**

(72) Erfinder: **Förster, Heinz, Dr, Am Eckbusch 47, D-5600 Wuppertal 1 (DE)**
Erfinder: **Eue, Ludwig, Dr., Paul-Klee-Strasse 36, D-5090 Leverkusen 1 (DE)**
Erfinder: **Schmidt, Robert Rudolf, Dr., Hahnenweg 5, D-5000 Köln 80 (DE)**

EP 0 014 900 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Phenoxycarbonsäure-carbonylalkylester, Verfahren zu ihrer Herstellung und ihre Verwendung als Herbizide sowie als Defoliants und Desiccants.

Die Erfindung betrifft neue Phenoxycarbonsäure-carbonylalkylester, ein Verfahren zu ihrer Herstellung und ihre Verwendung als Herbizide sowie als Defoliants und Desiccants.

Es ist bereits bekannt, daß bestimmte Phenoxycarbonsäureester herbizide Eigenschaften besitzen. So können zum Beispiel 5-(2-Chlor-4-trifluor-methyl-phenoxy)-2-nitro-α-phenoxy-propionsäure-methylester und 5-(2-Chlor-4-tri-fluormethyl-phenoxy)-2-nitro-α-phenoxy-propionsäure-ethylester zur Unkrautbekämpfung verwendet werden (vgl. US-A-4 093 446 und DE-A-2 311 638). Die Wirksamkeit dieser Stoffe ist sowohl bei einem Einsatz nach dem Pre-emergence- als auch nach dem Post-emergence-Verfahren gut. Nachteilig ist jedoch, daß einige schwierig zu bekämpfende Unkräuter und Ungräser nicht immer voll erfaßt werden. Darüber hinaus ist die Verträglichkeit in einigen Kulturen, z. B. Getreide, nicht immer voll ausreichend.

Es wurden nun neue Phenoxycarbonsäure-carbonylalkylester der Formel

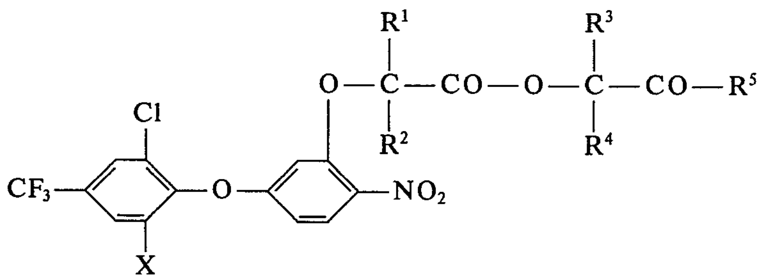

(I)

in welcher

$R^1$, $R^2$, $R^3$ und $R^4$ unabhängig von einander für Wasserstoff oder Alkyl stehen,

$R^5$ für Hydroxy, oder für gegebenenfalls durch Alkoxy, Alkylthio, Dialkylamino, Cyano oder Halogen substituiertes Alkoxy, für Alkenoxy, Alkinoxy, Aralkoxy oder Aryloxy steht oder für den Rest

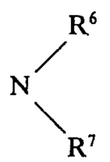

steht, worin

$R^6$ für Wasserstoff, Alkyl mit 1 bis 4 Kohlenstoffatomen, Alkenyl mit 3 bis 8 Kohlenstoffatomen, Benzyl oder gegebenenfalls durch Alkyl mit 1 bis 4 Kohlenstoffatomen, Alkoxy mit 1 bis 4 Kohlenstoffatomen, Alkylthio mit 1 bis 4 Kohlenstoffatomen, Alkoxycarbonyl mit 1 bis 4 Kohlenstoffatomen in der Alkoxygruppe, Fluor, Chlor, Brom, Trifluormethyl und/oder Nitro substituiertes Phenyl steht, und

$R^7$ für Alkyl mit 1 bis 4 Kohlenstoffatomen, Alkenyl mit 3 bis 8 Kohlenstoffatomen, Benzyl oder für gegebenenfalls durch Alkyl mit 1 bis 4 Kohlenstoffatomen, Alkoxy mit 1 bis 4 Kohlenstoffatomen, Alkylthio mit 1 bis 4 Kohlenstoffatomen, Alkoxycarbonyl mit 1 bis 4 Kohlenstoffatomen in der Alkoxygruppe, Fluor, Chlor, Brom, Trifluormethyl und/oder Nitro substituiertes Phenyl steht, oder

$R^6$ und $R^7$ zusammen dem Stickstoffatom, an das sie gebunden sind, für jeweils gegebenenfalls durch 1 oder 2 Methylgruppen substituiertes Pyrrolidinyl oder Morpholinyl, für jeweils gegebenenfalls durch 1 bis 3 Methyl- oder Ethylgruppen substituiertes Piperidinyl, Indolyl, Tetrahydroindolyl, Perhydroindolyl, Tetrahydrochinolyl, Tetrahydroisochinolyl, Perhydrochinolyl, Perhydroisochinolyl, Perhydrothiazolyl oder Perhydroazepinyl stehen, und

X für Wasserstoff oder Chlor steht,

gefunden.

Weiterhin wurde gefunden, daß man Phenoxycarbonsäurecarbonylalkylester der Formel (I) erhält, wenn man Phenoxycarbonsäurechloride der Formel

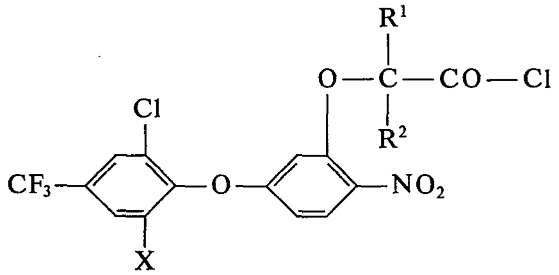

(II)

in welcher
$R^1$, $R^2$ und X die oben angegebene Bedeutung haben,
mit α-Hydroxy-carbonsäurederivaten der Formel

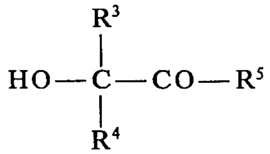

(III)

in welcher
$R^3$, $R^4$ und $R^5$ die oben angegebene Bedeutung haben,
gegebenenfalls in Gegenwart eines Säureakzeptors und gegebenenfalls in Gegenwart eines Verdünnungsmittels umsetzt.

Schließlich wurde gefunden, daß die neuen Phenoxycarbonsäure-carbonylalkylester der Formel (I) starke herbizide Eigenschaften besitzen und auch als Defoliants und Desiccants sehr gut verwendbar sind.

Überraschenderweise zeigen die erfindungsgemäßen Phenoxycarbonsäure-carbonylalkylester eine wesentlich bessere herbizide Wirkung als die aus dem Stand der Technik bekannten Verbindungen 5-(2-Chlor-4-trifluormethyl-phenoxy)-2-nitro-α-phenoxy-propionsäure-methylester und 5-(2-Chlor-4-trifluormethyl-phenoxy)-2-nitro-α-phenoxy-propionsäure-ethylester, welches die chemisch nächstliegenden Stoffe gleicher Wirkungsrichtung sind. Vorteilhaft ist vor allem, daß sich die erfindungsgemäßen Stoffe insbesondere bei einer Anwendung nach dem Pre-emrgence-Verfahren besser zur Vernichtung von schwierig zu bekämpfenden Unkräuter und Ungräsern, wie Galium und Cyperus, eignen als die genannten vorbekannten Stoffe. Außerdem zeichnen sich die erfindungsgemäßen Wirkstoffe bei einer Post-emergence-Applikation durch eine bessere Verträglichkeit bei Getreide aus als die erwähnten vorbekannten Stoffe.

Die erfindungsgemäßen Phenoxycarbonsäure-carbonylalkylester sind durch die Formel (I) allgemein definiert. In dieser Formel stehen $R^1$, $R^2$, $R^3$ und $R^4$ unabhängig voneinander vorzugsweise für Wasserstoff oder Methyl, $R^5$ steht vorzugsweise für Hydroxy, für gegebenenfalls durch $C_1-C_4$-Alkoxy, $C_1-C_4$-Alkylthio, Di-$C_1-C_4$-alkylamino, Cyano oder Chlor substituiertes $C_1-C_4$-Alkoxy, für $C_3-C_8$-Alkenoxy, $C_3-C_6$-Alkinoxy, Benzyloxy oder Phenoxy oder für den Rest

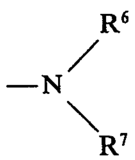

steht, worin
$R^6$ und $R^7$ diejenigen Bedeutungen haben, die oben bereits genannt wurden. X steht für Wasserstoff oder Chlor.

Verwendet man 5-(2,6-Dichlor-4-trifluormethyl-phenoxy)-2-nitro-α-phenoxy-propionsäure-chlorid und Hydroxyessigsäuremethylester als Ausgangsstoffe, so kann der Verlauf des erfindungsgemäßen Verfahrens durch das folgende Formelschema wiedergegeben werden:

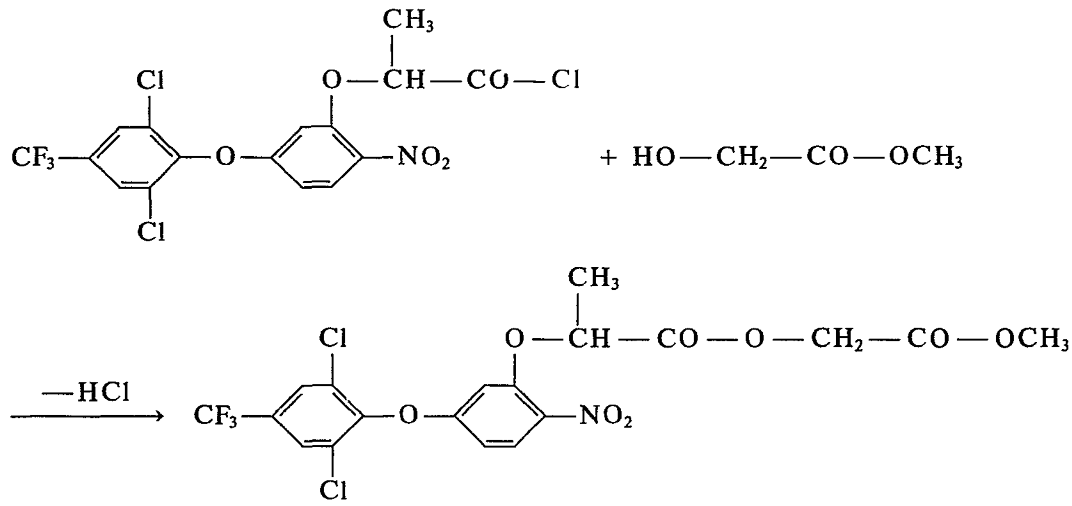


Die bei der Durchführung des erfindungsgemäßen Verfahrens als Ausgangsstoffe benötigten Phenoxycarbonsäurechloride sind durch die Formel (II) allgemein definiert. In dieser Formel stehen $R^1$, $R^2$ und X vorzugsweise für diejenigen Reste, die bereits im Zusammenhang mit der Beschreibung der erfindungsgemäßen Stoffe der Formel (I) vorzugsweise für diese Reste genannt werden.

Als Beispiele für die Phenoxycarbonsäurechloride der Formel (II) seien genannt: 5-(2-Chlor-4-trifluormethyl-phenoxy)-2-nitro-α-phenoxy- und 5-(2,6-Dichlor-4-trifluormethyl-phenoxy)-2-nitro-α-phenoxy-propionsäurechlorid.

Die Phenoxycarbonsäurechloride der Formel (II) sind bekannt oder lassen sich nach üblichen Methoden herstellen (vgl. US-A-4 093 446). Die bisher noch nicht bekannten Phenoxycarbonsäurechloride lassen sich synthetisieren, indem man Phenoxycarbonsäuren der Formel

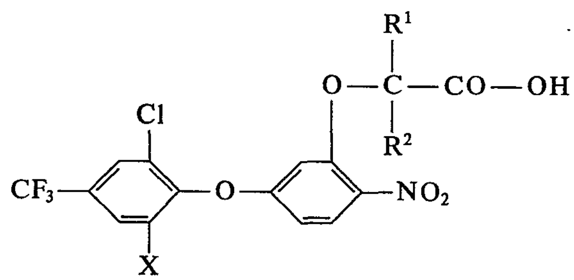
(IV)

in welcher
$R^1$, $R^2$ und X die oben angegebene Bedeutung haben,
mit Chlorierungsmitteln wie z. B. Thionylchlorid, gegebenenfalls unter Verwendung von Verdünnungsmitteln wie z. B. Benzol oder Ethylenchlorid, bei Temperaturen zwischen 10 und 100°C umsetzt und destillativ leicht flüchtige Komponenten nach dem Ende der Reaktion entfernt.

Die Phenoxycarbonsäuren der Formel (IV) sind ebenfalls bekannt oder lassen sich nach üblichen Verfahren herstellen (vgl. US-A-4 093 446). So erhält man die Verbindungen der Formel (IV), indem man Phenoxycarbonsäureester der Formel

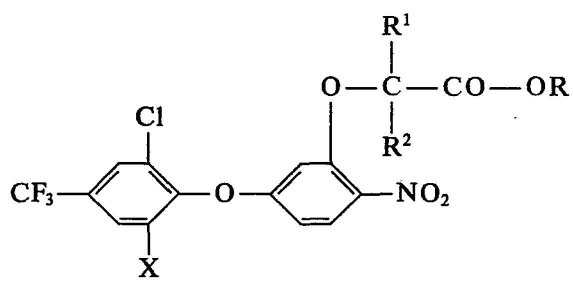
(V)

in welcher
$R^1$, $R^2$ und X die oben angegebene Bedeutung haben und
R für Alkyl (insbesondere für Methyl oder Ethyl) steht, mit wäßrigen Alkalihydroxidlaugen, vorzugsweise mit Natronlauge oder Kalilauge, welche gegebenenfalls mit organischen Lösungsmitteln wie z. B. Methanol, Ethanol oder Dioxan verdünnt sind, bei Temperaturen zwischen 20 und 100°C umsetzt.

Als Beispiele für die Phenoxycarbonsäuren der Formel (V) seien genannt:
5-(2-Chlor-4-trifluormethyl-phenoxy)-2-nitro-$\alpha$-phenoxy- und 5-(2,6-Dichlor-4-trifluormethyl-phenoxy)-2-nitro-$\alpha$-phenoxy-propionsäure.

Auch die Phenoxycarbonsäureester der Formel (V) sind bekannt oder lassen sich nach üblichen Verfahren herstellen (vgl. US-A-4 093 446). So erhält man die Verbindungen der Formel (V), indem man Phenol-Derivate der Formel

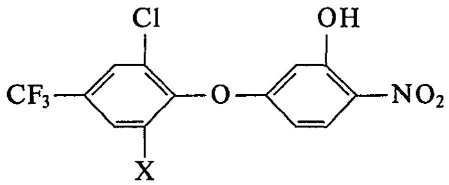
(VI)

in welcher
X die oben angegebene Bedeutung hat,
bzw. deren Natrium- oder Kaliumsalze,
mit $\alpha$-Halogen-carbonsäureestern der Formel

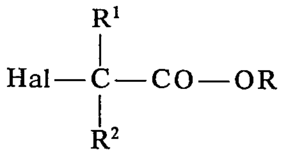
(VII)

in welcher
$R^1$ und $R^2$ die oben angegebene Bedeutung haben,
R für Alkyl (insbesondere für Methyl oder Ethyl) steht und
Hal für Chlor oder Brom steht,
gegebenenfalls in Gegenwart eines Säurebindemittels wie z. B. Natriummethylat oder Kaliumcarbonat und gegebenenfalls unter Verwendung eines polaren Verdünnungsmittels, wie z. B. Methanol, Acetonitril oder Sulfolan, bei Temperaturen zwischen 20 und 100° C umsetzt.

Als Beispiele für die Phenoxycarbonsäureester der Formel (V) seien genannt:
5-(2-Chlor-4-trifluormethyl-phenoxy)-2-nitro-$\alpha$-phenoxy- und 5-(2,6-Dichlor-4-trifluormethylphenoxy)-2-nitro-$\alpha$-phenoxy-propionsäuremethylester und -ethylester.

Die Phenolderivate der Formel (VI) sind bekannt (vergleiche US-A-4 093 446). Als Beispiele für die Phenolderivate der Formel (VI) seien genannt:
5-(2-Chlor-4-trifluormethyl-phenoxy)-2-nitro-phenol und 5-(2,6-Dichlor-4-trifluormethyl-phenoxy)-2-nitrophenol.

Bekannt sind auch die $\alpha$-Halogen-carbonsäureester der Formel (VII). Als Beispiele hierfür seien genannt: $\alpha$-Chlor-propionsäuremethylester und -ethylester sowie $\alpha$-Brom-propionsäuremethylester und -ethylester.

Die bei der Durchführung des erfindungsgemäßen Verfahrens weiterhin als Ausgangsstoffe benötigten $\alpha$-Hydroxycarbonsäure-Derivate sind durch die Formel (III) allgemein definiert. In dieser Formel stehen $R^3$, $R^4$ und $R^5$ vorzugsweise für diejenigen Reste, die bereits im Zusammenhang mit der Beschreibung der erfindungsgemäßen Stoffe der Formel (I) vorzugsweise für diese Reste genannt wurden.

Die $\alpha$-Hydroxy-carbonsäure-Derivate der Formel (III) sind bekannt oder lassen sich nach üblichen Verfahren herstellen (vgl. DE-A-2 201 432).

Die zum Teil noch nicht bekannten $\alpha$-Hydroxy-carbonsäureamide der Formel

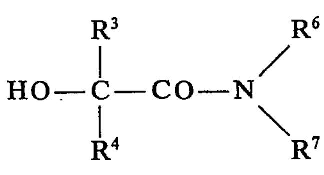
(IIIa)

in welcher
$R^3$, $R^4$, $R^6$ und $R^7$ die oben angegebene Bedeutung haben,
erhält man beispielsweise, indem man $\alpha$-Halogen-carbonsäureamide der Formel

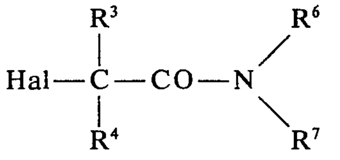

(VIII)

in welcher
$R^3$, $R^4$, $R^6$ und $R^7$ die oben angegebene Bedeutung haben und
Hal für Chlor oder Brom steht,
in einer ersten Stufe mit überschüssigem Natrium- oder Kaliumacetat, gegebenenfalls in Gegenwart eines Katalysators wie z. B. Tetrabutylammoniumbromid und gegebenenfalls unter Verwendung eines Verdünnungsmittels wie z. B. Essigsäure oder Toluol, bei Temperaturen zwischen 20 und 200°C umsetzt und in einer zweiten Stufe die dabei entstehenden Acetoxycarbonsäureamide der Formel

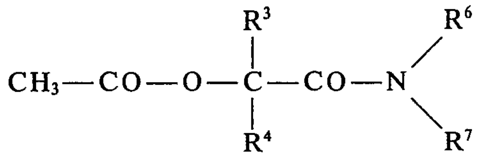

(IX)

in welcher
$R^3$, $R^4$, $R^6$ und $R^7$ die oben angegebene Bedeutung haben, durch Umsetzung mit verdünnter wäßrig-alkoholischer Natronlauge bei Temperaturen zwischen 20 und 150°C entacyliert.

Als Beispiele für die α-Hydroxy-carbonsäurederivate der Formel (III) seien genannt: Hydroxy-essigsäure-, α-Hydroxy-propionsäure- und α-Hydroxy-iso-buttersäuremethylester, -ethylester, -n-propylester, -iso-propylester, -n-butylester, -iso-butylester, -sek.-butylester, -tert.-butylester, benzylester, -phenylester, -methylamid, -ethylamid, -n-propylamid, -iso-propylamid, -n-butylamid, -iso-butylamid, -dimethylamid, -diethylamid, -di-n-propylamid, -di-isopropylamid, -N-methyl-N-iso-propylamid, -N-methyl-N-iso-butylamid, -N-methyl-N-sek.-butylamid, -anilid, -2-nitro-, 3-nitro- und -4-nitro-phenylamid, -2-chlor-, -3-chlor- und 4-chlorphenylamid, -2,4-dichlor-, -2,5-dichlor-, -3,4-dichlor- und -3,5-dichlor-phenylamid, -2-methyl-, -3-methyl- und -4-methyl-phenylamid, -N-methyl-anilid, -N-methyl-N-(2-methyl-phenyl)-amid, -N-methyl-N-(2-nitrophenyl)-, -N-methyl-N-(3-nitrophenyl)-, -N-methyl-N-(4-nitrophenyl)-amid, -N-methyl-N-(2-chlorphenyl)-, -N-methyl-N-(3-chlorphenyl)-, -N-methyl-N-(4-chlorphenyl)-amid, -N-methyl-N-(3-nitro-6-methyl-phenyl)-amid, -N-ethyl-anilid, -N-ethyl-N-(2-nitro-phenyl)-, -N-ethyl-N-(3-nitro-phenyl)-, -N-ethyl-N-(4-nitro-phenyl)-amid, -N-ethyl-N-(2-chlor-phenyl)-, -N-ethyl-N-(3-chlorphenyl)-, -N-ethyl-N-(4-chlor-phenyl)-amid, -N-ethyl-N-(3-nitro-6-methyl-phenyl)-amid, -N-propyl-anilid, -N-propyl-N-(2-nitro-phenyl)-, -N-propyl-N-(3-nitro-phenyl)-, -N-propyl-N-(4-nitro-phenyl)-amid, -N-propyl-N-(2-chlor-phenyl)-, -N-propyl-N-(3-chlor-phenyl)-, -N-propyl-N-(4-chlor-phenyl-amid, -N-propyl-N-(2-methyl-phenyl)-, -N-propyl-N-(3-methyl-phenyl-, -N-propyl-N-(4-methyl-phenyl)-amid, -N-propyl-N-(3-nitro-6-methyl-phenyl)-amid, -N-butyl-anilid, -N-butyl-N-(2-nitro-phenyl)-, -N-butyl-N-(3-nitro-phenyl)-, -N-butyl-N-(4-nitro-phenyl)-amid, -N-butyl-N-(2-chlor-phenyl)-, -N-butyl-N-(3-chlor-phenyl)-, -N-butyl-N-(4-chlor-phenyl)-amid, -N-butyl-N-(2-methyl-phenyl)-, -N-butyl-N-(3-methyl-phenyl)-, -N-butyl-N-(4-methyl-phenyl)-amid, -N-butyl-N-(3-nitro-6-methyl-phenyl)-amid, -N-isobutylanilid, -N-iso-butyl-N-(2-nitro-phenyl)-, -N-iso-butyl-N-(3-nitro-phenyl-, -N-iso-butyl-N-(4-nitro-phenyl)-amid, -N-iso-butyl-N-(2-chlor-phenyl)-, -N-iso-butyl-N-(3-chlor-phenyl)-, -N-iso-butyl-N-(4-chlor-phenyl)-amid, -N-iso-butyl-N-(2-methyl-phenyl)-, -N-iso-butyl-N-(3-methyl-phenyl)-, -N-iso-butyl-N-(4-methyl-phenyl)-amid, -N-iso-butyl-N-(3-nitro-6-methyl-phenyl)-amid, -benzylamid, -dibenzylamid, -N-methyl-N-butylamid, -N-ethyl-N-benzylamid, -N-propyl-N-benzylamid, -N-butyl-N-benzylamid, -pyrrolidid, -2-methyl-pyrrolidid, -morpholid, -piperidid, -2-methyl-piperidid, -4-methyl-piperidid, -2,4-dimethyl-piperidid, -2,4,6-trimethyl-piperidid, -2-ethyl-piperidid, -4-ethyl-piperidid, -2,4-diethyl-piperidid, -2,4,6-triethyl-piperidid, -2-methyl-4-ethyl-piperidid, -2-ethyl-4-methyl-piperidid, -2-methyl-5-ethyl-piperidid, -2-ethyl-5-methyl-piperidid, -2-methyl-6-ethyl-piperidid, -1,2,3,4-tetrahydroindolid, -2-methyl-1,2,3,4-tetrahydroindolid, -perhydroindolid, -2-methyl-perhydroindolid, -2,2-dimethyl-perhydroindolid, -1,2,3,4-tetrahydrochinolid, -2-methyl-1,2,3,4-tetrahydrochinolid, -perhydrochinolid, -2-methyl-perhydrochinolid, -4-methyl-perhydrochinolid, -1,2,3,4-tetrahydroiso-chinolid und -perhydroisochinolid.

Die α-Halogencarbonsäureamide der Formel (VIII) sind bekannt oder können analog bekannten Verfahren hergestellt werden. Man erhält z. B. durch Umsetzung von α-Halogencarbonsäurehalogeniden wie z. B. Chloracetylchlorid mit Ammoniak bzw. primären oder sekundären Aminen, gegebenenfalls in Gegenwart eines Säureakzeptors wie z. B. Kaliumhydroxid (vergleiche J. Agric. Food Chem. 4 (1956), 518—522).

Als Beispiele für die Halogencarbonsäureamide der Formel (VIII) seien genannt: Chloressigsäure-, α-Chlorpropionsäure- und -α-Chloroisobuttersäure-methylamid, -ethylamid, -n-propyl-amid, -iso-propylamid, -n-butylamid, -iso-butylamid, -dimethyl-amid, -diethylamid, -di-n-propylamid, -di-iso-propylamid, -N-methyl-N-iso-propylamid, -N-methyl-N-isobutylamid, -N-methyl-N-sek.-

butylamid, -anilid, -2-nitro-, -3-nitro- und 4-nitro-phenylamid, -2-chlor-, -3-chlor- und -4-chlorphenylamid, -2,4-dichlor-, -2,5-dichlor-, -3,4-dichlor- und -3,5-dichlor-phenylamid, -2-methyl-, -3-methyl- und -4-methyl-phenylamid, -N-methyl-anilid, -N-methyl-N-(2-methyl-phenyl)-amid, -N-methyl-N-(2-nitro-phenyl)-, -N-methyl-N-(3-nitro-phenyl)-, -N-methyl-N-(4-nitro-phenyl)-amid, -N-methyl-N-(2-chlor-phenyl)-, -N-methyl-N-(3-chlor-phenyl)-, -N-methyl-N-(4-chlor-phenyl)-amid, -N-methyl-N-(3-nitro-6-methyl-phenyl)-amid, -N-ethyl-anilid, -N-ethyl-N-(2-nitrophenyl)-, -N-ethyl-N-(3-nitro-phenyl)-, -N-ethyl-N-(4-nitro-phenyl)-amid, -N-ethyl-N-(2-chlor-phenyl)-, -N-ethyl-N-(3-chlor-phenyl)-, -N-ethyl-N-(4-chlor-phenyl)-amid, -N-ethyl-N-(3-nitro-6-methyl-phenyl)-amid, -N-propyl-anilid, -N-propyl-N-(2-nitro-phenyl)-, -N-propyl-N-(3-nitro-phenyl)-, -N-propyl-N-(4-nitro-phenyl)-amid, -N-propyl-N-(2-chlor-phenyl)-, -N-propyl-N-(3-chlor-phenyl)-, -N-propyl-N-(4-chlor-phenyl)-amid, -N-propyl-N-(2-methyl-phenyl)-, -N-propyl-N-(3-methyl-phenyl)-, -N-propyl-N-(4-methyl-phenyl)-amid, -N-propyl-N-(3-nitro-6-methyl-phenyl)-amid, -N-butyl-anilid, -N-butyl-N-(2-nitro-phenyl)-, -N-butyl-N-(3-nitro-phenyl)-, -N-butyl-N-(4-nitro-phenyl)-amid, -N-butyl-N-(2-chlor-phenyl)-, -N-butyl-N-(3-chlor-phenyl)-, -N-butyl-N-(4-chlor-phenyl)-amid, -N-butyl-N-(2-methyl-phenyl)-, -N-butyl-N-(3-methyl-phenyl)-, -N-butyl-N-(4-methyl-phenyl)-amid, -N-butyl-N-(3-nitro-6-methyl-phenyl)-amid, -N-isobutyl-anilid, -N-iso-butyl-N-(2-nitro-phenyl)-, -N-iso-butyl-N-(3-nitro-phenyl)-, -N-iso-butyl-N-(4-nitro-phenyl)-amid, -N-iso-butyl-N-(2-chlor-phenyl)-, -N-iso-butyl-N-(3-chlor-phenyl)-, -N-iso-butyl-N-(4-chlor-phenyl)-amid, -N-iso-butyl-N-(2-methyl-phenyl)-, -N-iso-butyl-N-(3-methyl-phenyl)-, -N-iso-butyl-N-(4-methyl-phenyl)-amid, -N-iso-butyl-N-(3-nitro-6-methyl-phenyl)-amid, -benzylamid, -dibenzylamid, -N-methyl-N-benzyl-amid, -N-ethyl-N-benzylamid, -N-propyl-N-benzylamid, -N-butyl-N-benzylamid, -pyrrolidid, -2-methyl-pyrrolidid, -morpholid, -piperidid, -2-methyl-piperidid, -4-methyl-piperidid, -2,4-dimethyl-piperidid, -2,4,6-trimethyl-piperidid, -2-ethyl-piperidid, -4-ethyl-piperidid, 2,4-diäthylpiperidid, -2,4,6-triethyl-piperidid, -2-methyl-4-ethyl-piperidid, -2-ethyl-4-methyl-piperidid, -2-methyl-5-ethyl-piperidid, -2-ethyl-5-methyl-piperidid, -2-methyl-6-ethyl-piperidid, -1,2,3,4-tetrahydroindolid, -2-methyl-1,2,3,4-tetrahydroindolid, -perhydroindolid, -2-methyl-perhydroindolid, -2,2-dimethyl-perhydroindolid, -1,2,3,4-tetrahydrochinolid, -2-methyl-1,2,3,4-tetrahydrochinolid, -perhydrochinolid, -2-methyl-perhydrochinolid, -4-methyl-perhydrochinolid, -1,2,3,4-tetrahydroisochinolid und -perhydroisochinolid.

Das erfindungsgemäße Verfahren wird bevorzugt unter Verwendung geeigneter Lösungs- oder Verdünnungsmittel durchgeführt. Als solche kommen praktisch alle inerten organischen Solventien in Frage.

Hierzu gehören insbesondere aliphatische und aromatische, gegebenenfalls chlorierte Kohlenwasserstoffe, wie Benzin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Tetrahydrofuran und Dioxan, Ketone wie Aceton, Methylethyl-, Methylisopropyl- und Methylisobutylketon, Nitrile wie Acetonitril und Propionitril sowie aprotisch polare Lösungsmittel wie z. B. Dimethylformamid, Dimethylsulfoxid, Sulfolan und Hexamethylphosphorsäuretriamid.

Als Säureakzeptoren können alle üblichen Säurebindemittel Verwendung finden. Besonders bewährt haben sich Alkalicarbonate und -alkoholate, wie Natrium- und Kaliumcarbonat, Natrium- und Kaliummethylat bzw. -ethylat, ferner aliphatische, aromatische oder heterocyclische Amine, beispielsweise Triethylamid, Trimethylamin, Dimethylanilin, Dimethylbenzylamin und Pyridin.

Die Reaktionstemperatur kann innerhalb eines größeren Bereichs variiert werden. Im allgemeinen arbeitet man zwischen −20 und 100° C, vorzugsweise zwischen 0 und 50° C.

Das erfindungsgemäße Verfahren wird im allgemeinen bei Normaldruck durchgeführt.

Zur Durchführung des erfindungsgemäßen Verfahrens setzt man auf 1 Mol Phenoxycarbonsäurechlorid der Formel (II) im allgemeinen 1 bis 1,5 Mol, vorzugsweise 1 bis 1,2 Mol $\alpha$-Hydroxy-carbonsäurederivat der Formel (III) sowie 1 bis 5 Mol Säurebindemittel ein. Die Umsetzung wird im allgemeinen in einem geeigneten Verdünnungsmittel durchgeführt und das Reaktionsgemisch wird mehrere Stunden bei der erforderlichen Temperatur gerührt. Danach gibt man ein organisches Lösungsmittel, z. B. Toluol zu, und arbeitet die organische Phase wie üblich durch Waschen, Trocknen und Abdestillieren des Lösungsmittels auf.

Die neuen Verbindungen fallen teilweise in Form von Ölen an, die sich zum Teil nicht unzersetzt destillieren lassen, jedoch durch sogenanntes »Andestillieren«, d. h. durch längeres Erhitzen unter vermindertem Druck auf mäßig erhöhte Temperaturen von den letzten flüchtigen Anteilen befreit und auf diese Weise gereinigt werden können. Zu ihrer Charakterisierung dient der Brechungsindex.

Soweit die Produkte in fester Form anfallen, können sie durch Umkristallisation gereinigt werden. Zur Charakterisierung dient dann der Schmelzpunkt.

Die erfindungsgemäßen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z. B. bei den folgenden Pflanzen verwendet werden:

Dikotyle Unkräuter der Gattungen:
Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dicotyle Kulturen der Gattungen:
Gossypium, Glycine, Beta, Daucus, Phaseolus, Pisum, Solanum, Linum, Ipomoea, Vicia, Nicotiana, Lycopersion, Arachis, Brassica, Lactuca, Cucumis, Cuburbita.

Monokotyle Unkräuter der Gattungen:
Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen:
Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z. B. auf Industrie- und Gleisanlagen und auf Wegen und Plätzen mit und ohne Baumbewuchs. Ebenso können die Verbindungen zur Unkrautbekämpfung in Dauerkulturen z. B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe zeigen eine sehr gute Wirkung gegen Unkräuter und Ungräser. Sie lassen sich insbesondere zur Beseitigung von schwierig zu bekämpfenden Unkräutern und Ungräsern, wie Galium und Cyperus, verwenden. Zur weiteren Verbesserung des Wirkungsspektrums sind Kombinationen mit anderen Herbiziden möglich, vor allem mit N-Benzthiazol-2-yl-N,N'-dimethylharnstoff.

Die Wirkstoffe können in die üblichen Formulierungen übergeführt werden, wie Lösungen, Emulsionen, Suspensionen, Pulver, Schäume, Pasten, Granulate, Wirkstoffimprägnierte Natur- und synthetische Stoffe und Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z. B. nichtionogene und anionische Emulgatoren, wie Polyoxyäthylen-Fettsäure-Ester, Polyoxyäthylen-Fettalkohol-Äther, z. B. Alkylaryl-polyglykol-äther, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90%.

Die erfindungsgemäßen Wirkstoffe können als solche oder in ihren Formulierungen auch in

Mischung mit bekannten Herbiziden zur Unkrautbekämpfung Verwendung finden, wobei Fertigformulierung oder Tankmischung möglich ist. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die Wirkstoffe können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z. B. durch Gießen, Spritzen, Sprühen, Streuen.

Die erfindungsgemäßen Wirkstoffe können sowohl vor als auch nach dem Auflaufen der Pflanzen appliziert werden. Die Anwendung wird vorzugsweise vor dem Auflaufen der Pflanzen, also im pre-emergence-Verfahren, vorgenommen. Sie können auch vor der Saat in den Boden eingearbeitet werden.

Die aufgewandte Wirkstoffmenge kann in größeren Bereichen schwanken. Sie hängt im wesentlichen von der Art des gewünschten Effekts ab. Im allgemeinen liegen die Aufwandmengen zwischen 0,05 und 10 kg Wirkstoff pro ha, vorzugsweise zwischen 0,1 und 5 kg/ha.

Die gute herbizide Wirksamkeit der erfindungsgemäßen Verbindungen geht aus den nachfolgenden Beispielen hervor.

In diesen Beispielen werden die nachstehend angegebenen Stoffe als Vergleichssubstanzen eingesetzt:

(A) = 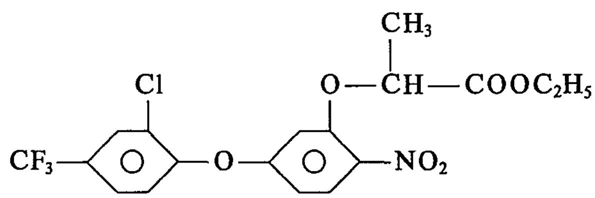

(B) = 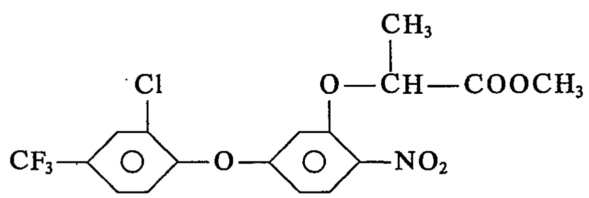

Beispiel A

Pre-emergence-Test

Lösungsmittel: 5 Gewichtsteile Aceton
Emulgator: 1 Gewichtsteil Alkylarylpolyglycoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)
100% = totale Vernichtung

In diesem Test zeigt die im Beispiel (14) beschriebene erfindungsgemäße Verbindung eine wesentlich bessere Wirksamkeit gegen Cyperus als die bekannte Vergleichssubstanz (A).

Beispiel B

Pre-emergence-Test

Lösungsmittel: 5 Gewichtsteile Aceton
Emulgator: 1 Gewichtsteil Alkylarylpolyglycoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)
100% = totale Vernichtung

In diesem Test zeigen die in den Beispielen (2) und (13) beschriebenen erfindungsgemäßen Verbindungen eine wesentlich bessere Wirkung gegen Galium als die bekannte Vergleichssubstanz (B).

Beispiel C

Post-emergence-Test

Lösungsmittel: 5 Gewichtsteile Aceton
Emulgator: 1 Gewichtsteil Alkylarylpolyglycoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5–15 cm haben so, daß die jeweils gewünschte Wirkstoffmenge pro Flächeneinheit ausgebracht wird. Die Konzentration der Spritzbrühe wird so gewählt, daß in 2000 l Wasser/ha die jeweils gewünschte Wirkstoffmenge ausgebracht wird. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)
100% = totale Vernichtung

In diesem Test zeigt die in dem Beispiel (14) beschriebene erfindungsgemäße Verbindung bei gleich guter Wirkung gegen Stellaria und Matricaria eine wesentlich bessere Verträglichkeit bei Weizen als die bekannte Vergleichssubstanz (A).

Beispiel D

Entlaubung und Austrocknung der Blätter bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des fünften Folgeblattes angezogen. In diesem Stadium werden die Pflanzen tropfnaß mit den Wirkstoffzubereitungen besprüht. Nach einer Woche werden der Blattfall und das Austrocknen der Blätter im Vergleich zu den Kontrollpflanzen bonitiert. Es bedeuten:

O kein Austrocknen der Blätter, kein Blattfall
+ leichtes Austrocknen der Blätter, geringer Blattfall
++ starkes Austrocknen der Blätter, starker Blattfall
+++ sehr starkes Austrocknen der Blätter, sehr starker Blattfall

In diesem Test zeigen die in den Beispielen (5), (12) und (14) beschriebenen Verbindungen eine sehr gute Wirksamkeit.

## Beispiel E

### Wuchshemmung bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des fünften Folgeblattes angezogen. In diesem Stadium werden die Pflanzen tropfnaß mit den Wirkstoffzubereitungen besprüht. Nach 3 Wochen wird der Zuwachs der Pflanzen gemessen und die Wuchshemmung in Prozent des Zuwachses der Kontrollpflanzen berechnet. Es bedeuten 100% Wuchshemmung den Stillstand des Wachstums und 0% ein Wachstum entsprechend dem der Kontrollpflanzen.

In diesem Test zeigt die in dem Beispiel (12) beschriebene Verbindung eine sehr starke wuchshemmende Wirksamkeit.

## Herstellungsbeispiele

### Beispiel 1

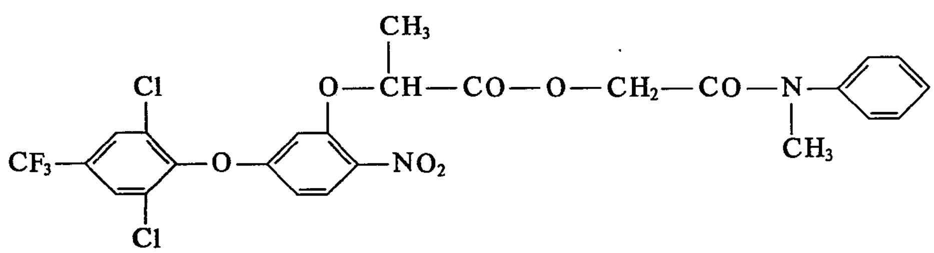

Eine Lösung von 12,5 g (27 mMol) 5-(2,6-Dichlor-4-trifluormethyl-phenoxy)-2-nitro-α-phenoxy-propionsäurechlorid in 30 ml Toluol wird zu einer auf 0 bis 5°C gekühlten Lösung von 5 g (30 mMol) Hydroxy-essigsäure-N-methyl-anilid und 10 ml Pyridin in 70 ml Toluol tropfenweise gegeben. Das Reaktionsgemisch wird über Nacht bei Raumtemperatur gerührt, mit 300 ml Toluol verdünnt, neutral gewaschen, getrocknet, filtriert und eingeengt. Man erhält 13 g (81% der Theorie) 5-(2,6-Dichlor-4-trifluormethyl-phenoxy)-2-nitro-α-phenoxy-propionsäure-(N-methyl-N-phenyl-carbamoyl-methyl)-ester in Form eines gelbstichigen Öles vom Brechungsindex $n_D^{24}$: 1,5607.

Analog Beispiel 1 werden die in der folgenden Tabelle 1 aufgeführten Verbindungen der Formel (I) hergestellt:

Tabelle 1

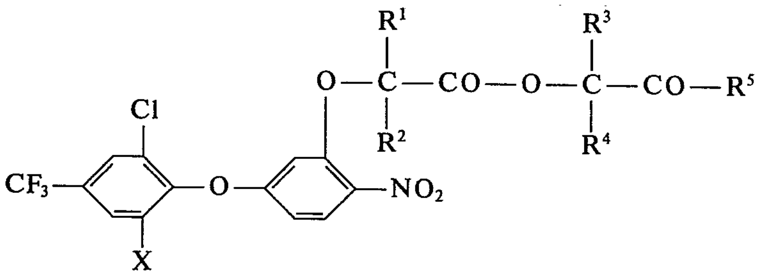

(I)

| Beispiel Nr. | X | $R^1$ | $R^2$ | $R^3$ | $R^4$ | $R^5$ | Schmelzpunkt (°C); Brechungsindex: |
|---|---|---|---|---|---|---|---|
| 2 | H | H | $CH_3$ | H | H | $-OC_4H_9$-n | $n_D^{24}$: 1,5227 |
| 3 | H | H | $CH_3$ | H | H | 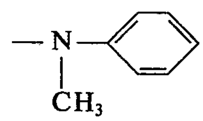 | $n_D^{24}$: 1,5606 |
| 4 | H | H | H | H | H | $-OC_2H_5$-n | 88 |
| 5 | H | H | $CH_3$ | H | H | $-N(CH_3)_2$ | $n_D^{24}$: 1,5235 |
| 6 | H | H | $CH_3$ | H | H | $-N(CH_2-CH=CH_2)_2$ | $n_D^{24}$: 1,5214 |
| 7 | H | H | $CH_3$ | H | H | 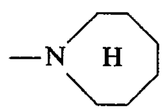 | $n_D^{24}$: 1,5132 |
| 8 | H | H | $CH_3$ | H | H | 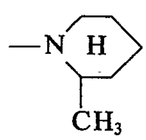 | $n_D^{25}$: 1,5215 |
| 9 | H | H | $CH_3$ | H | H | 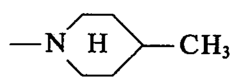 | $n_D^{24}$: 1,5312 |
| 10 | H | H | $CH_3$ | H | $CH_3$ | 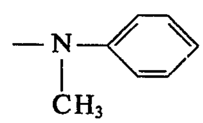 | $n_D^{24}$: 1,5760 |
| 11 | H | H | $CH_3$ | H | $CH_3$ | $-OC_2H_5$ | $n_D^{21}$: 1,5221 |
| 12 | Cl | H | $CH_3$ | H | H | $-OC_4H_9$-n | $n_D^{24}$: 1,5606 |
| 13 | H | H | $CH_3$ | H | H | $-OC_2H_5$ | $n_D^{24}$: 1,5297 |
| 14 | Cl | H | $CH_3$ | H | H | $-OC_2H_5$ | $n_D^{24}$: 1,5330 |
| 15 | Cl | H | $CH_3$ | H | $CH_3$ | $-OC_2H_5$ | 74 |
| 16 | Cl | H | $CH_3$ | H | H | 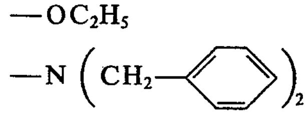 | 44 |
| 17 | Cl | H | $CH_3$ | H | H | 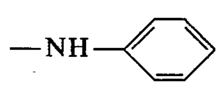 | 60 |
| 18 | Cl | H | $CH_3$ | H | H | 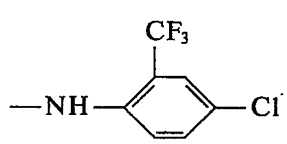 | 46 |

Fortsetzung

| Beispiel Nr. | X | $R^1$ | $R^2$ | $R^3$ | $R^4$ | $R^5$ | Schmelzpunkt (°C); Brechungsindex: |
|---|---|---|---|---|---|---|---|
| 19 | Cl | H | $CH_3$ | H | $CH_3$ | 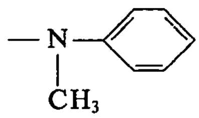 | 132 |
| 20 | Cl | H | $CH_3$ | $CH_3$ | $CH_3$ | $-OC_2H_5$ | $n_D^{22}$: 1,5319 |
| 21 | Cl | H | $CH_3$ | H | H | $-OCH_3$ | $n_D^{21,5}$: 1,542 |

Die als Ausgangsstoffe zu verwendenden Phenoxycarbonsäurechloride können beispielsweise wie folgt hergestellt werden:

Beispiel II-1

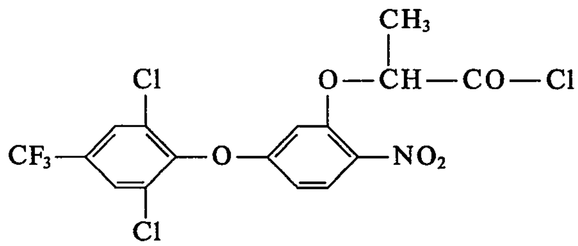

9,7 g (82 mMol) Thionylchlorid werden bei Raumtemperatur tropfenweise zu einer Lösung von 30 g (68 mMol) 5-(2,6-Dichlor-4-trifluormethyl-phenoxy)-2-nitro-$\alpha$-phenoxy-propionsäure und 0,5 Dimethylformamid in 100 ml 1,2-Dichlorethan gegeben. Die Mischung wird 4 Stunden unter Rückfluß erhitzt. Man gibt Aktivkohle dazu, filtriert und engt ein. Nach Digerieren des öligen Rückstandes mit 100 ml Ligroin erhält man 26,2 g (84% der Theorie) 5-(2,6-Dichlor-4-trifluor-methyl-phenoxy)-2-nitro-$\alpha$-phenoxy-propionsäurechlorid in Form gelber Kristalle vom Schmelzpunkt 92° C.

Die als Vorprodukte benötigten Phenoxycarbonsäuren können beispielsweise wie folgt hergestellt werden:

Beispiel IV-1

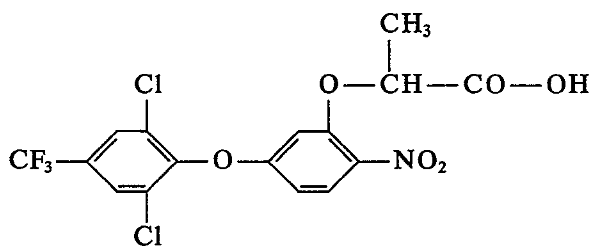

135 g (0,3 Mol) 5-(2,6-Dichlor-4-trifluormethyl-phenoxy)-2-nitro-$\alpha$-phenoxy-propionsäuremethylester und 30 ml konzentrierter Natronlauge werden in 400 ml Acetonitril und 150 ml Wasser 24 Stunden bei 20° C gerührt. Die Lösung wird eingeengt, der Rückstand in 500 ml Wasser aufgenommen und mit 50 ml konzentrierter Salzsäure angesäuert. Man erhält 93 g (71% der Theorie) 5-(2,6-Dichlor-4-trifluormethyl-phenoxy)-2-nitro-$\alpha$-phenoxy-propionsäure mit in Form blaßgelber Kristalle mit dem Schmelzpunkt 146° C (nach Umkristallisation aus Toluol/Cyclohexan).

Die als Vorprodukte benötigten Phenoxycarbonsäureester können wie folgt hergestellt werden:

Beispiel V-1

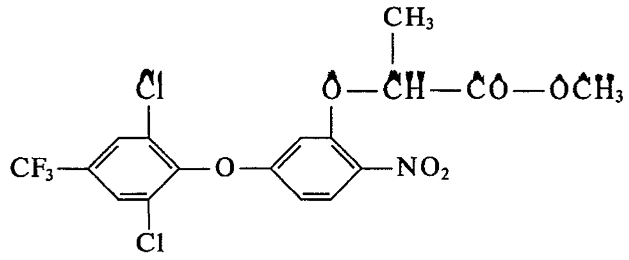

40 g (0,24 Mol) α-Brom-propionsäuremethylester werden tropfenweise zu einer auf 50° C erwärmten Mischung aus 73,6 g (0,2 Mol) 5-(2,6-Dichlor-4-trifluormethyl-phenoxy)-2-nitro-phenol, 32 g Kaliumcarbonat und 200 ml Acetonitril gegeben. Das Reaktionsgemisch wird 5 Stunden unter Rückfluß erhitzt, dann in 1 l Wasser gegossen und mit 1 l Toluol extrahiert. Die Toluolphase wird mit 300 ml in wäßriger Natronlauge und dann mit 500 ml Wasser gewaschen. Nach Abdestillieren des Lösungsmittels im Vakuum erhält man als Öl, welches bei Zugabe von Methanol kristallisiert, 74 g (81% der Theorie) 5-(2,6-Dichlor-4-trifluormethyl-phenoxy)-2-nitro-α-phenoxy-propionsäuremethylester mit dem Schmelzpunkt 78° C.

**Patentansprüche**

1. Phenoxycarbonsäure-carbonylalkylester der Formel

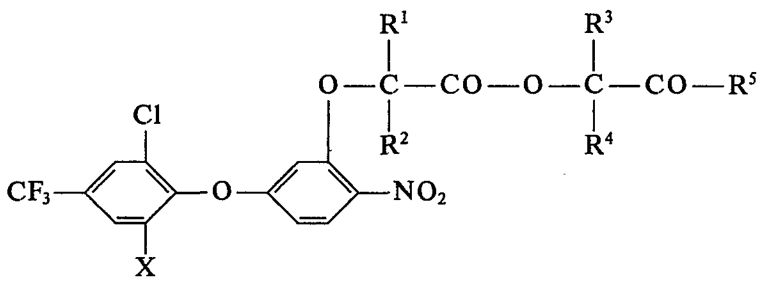

(I)

in welcher

$R^1$, $R^2$, $R^3$ und $R^4$ unabhängig voneinander für Wasserstoff oder Alkyl stehen,

$R^5$ für Hydroxy, oder für gegebenenfalls durch Alkoxy, Alkylthio, Dialkylamino, Cyano oder Halogen substituiertes Alkoxy, für Alkenoxy, Alkinoxy, Aralkoxy oder Aryloxy steht oder für den Rest

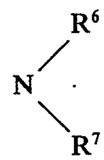

steht, worin

$R^6$ für Wasserstoff, Alkyl mit 1 bis 4 Kohlenstoffatomen, Alkenyl mit 3 bis 8 Kohlenstoffatomen, Benzyl oder gegebenenfalls durch Alkyl mit 1 bis 4 Kohlenstoffatomen, Alkoxy mit 1 bis 4 Kohlenstoffatomen, Alkylthio mit 1 bis 4 Kohlenstoffatomen, Alkoxycarbonyl mit 1 bis 4 Kohlenstoffatomen in der Alkoxygruppe, Fluor, Chlor, Brom, Trifluormethyl und/oder Nitro substituiertes Phenyl steht, und

$R^7$ für Alkyl mit 1 bis 4 Kohlenstoffatomen, Alkenyl mit 3 bis 8 Kohlenstoffatomen, Benzyl oder für gegebenenfalls durch Alkyl mit 1 bis 4 Kohlenstoffatomen, Alkoxy mit 1 bis 4 Kohlenstoffatomen, Alkylthio mit 1 bis 4 Kohlenstoffatomen, Alkoxycarbonyl mit 1 bis 4 Kohlenstoffatomen in der Alkoxygruppe, Fluor, Chlor, Brom, Trifluormethyl und/oder Nitro substituiertes Phenyl steht, oder

$R^6$ und $R^7$ zusammen mit dem Stickstoffatom, an das sie gebunden sind, für jeweils gegebenenfalls durch 1 oder 2 Methylgruppen substituiertes Pyrrolidinyl oder Morpholinyl, für jeweils gegebenenfalls durch 1 bis 3 Methyl- oder Ethylgruppen substituiertes Piperidinyl, Indolyl, Tetrahydroindolyl, Perhydroindolyl, Tetrahydrochinolyl, Tetrahydroisochinolyl, Perhydrochinolyl, Perhydroisochinolyl, Perhydrothiazolyl oder Perhydroazepinyl stehen, und

X steht für Wasserstoff oder Chlor.

2. Verfahren zur Herstellung von Phenoxycarbonsäurecarbonylalkylestern der Formel

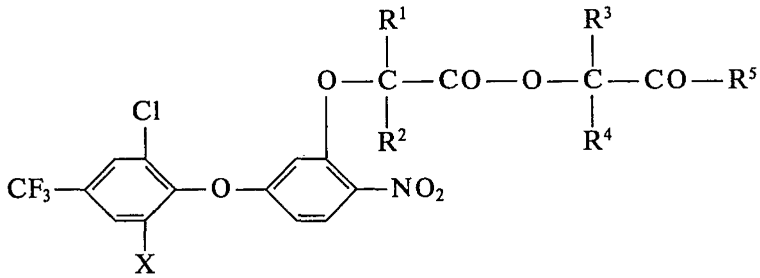

(I)

in welcher

$R^1$, $R^2$, $R^3$ und $R^4$ unabhängig voneinander für Wasserstoff oder Alkyl stehen,

$R^5$ für Hydroxy oder für gegebenenfalls durch Alkoxy, Alkylthio, Dialkylamino, Cyano oder Halogen substituiertes Alkoxy, für Alkenoxy, Alkinoxy, Aralkoxy oder Aryloxy steht oder für den Rest

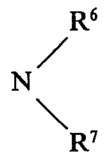

steht, worin

$R^6$ für Wasserstoff, Alkyl mit 1 bis 4 Kohlenstoffatomen, Alkenyl mit 3 bis 8 Kohlenstoffatomen, Benzyl oder gegebenenfalls durch Alkyl mit 1 bis 4 Kohlenstoffatomen, Alkoxy mit 1 bis 4 Kohlenstoffatomen, Alkylthio mit 1 bis 4 Kohlenstoffatomen, Alkoxycarbonyl mit 1 bis 4 Kohlenstoffatomen in der Alkoxygruppe, Fluor, Chlor, Brom, Trifluormethyl und/oder Nitro substituiertes Phenyl steht, und

$R^7$ für Alkyl mit 1 bis 4 Kohlenstoffatomen, Alkenyl mit 3 bis 8 Kohlenstoffatomen, Benzyl oder für gegebenenfalls durch Alkyl mit 1 bis 4 Kohlenstoffatomen, Alkoxy mit 1 bis 4 Kohlenstoffatomen, Alkylthio mit 1 bis 4 Kohlenstoffatomen, Alkoxycarbonyl mit 1 bis 4 Kohlenstoffatomen in der Alkoxygruppe, Fluor, Chlor, Brom, Trifluormethyl und/oder Nitro substituiertes Phenyl steht, oder

$R^6$ und $R^7$ zusammen mit dem Stickstoffatom, an das sie gebunden sind, für jeweils gegebenenfalls durch 1 oder 2 Methylgruppen substituiertes Pyrrolidinyl oder Morpholinyl, für jeweils gegebenenfalls durch 1 bis 3 Methyl- oder Ethylgruppen substituiertes Piperidinyl, Indolyl, Tetrahydroindolyl, Perhydroindolyl, Tetrahydrochinolyl, Tetrahydroisochinolyl, Perhydrochinolyl, Perhydroisochinolyl, Perhydrothiazolyl oder Perhydroazepinyl stehen, und

X steht für Wasserstoff oder Chlor,

dadurch gekennzeichnet, daß man Phenoxycarbonsäurechloride der Formel

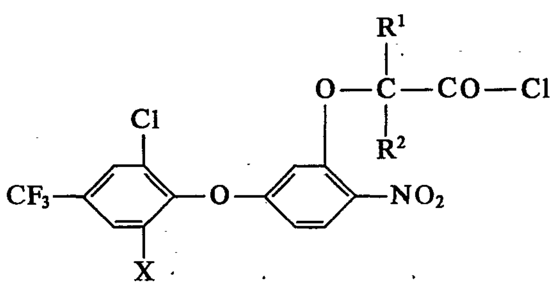

(II)

in welcher

$R^1$, $R^2$ und X die oben angegebene Bedeutung haben, mit $\alpha$-Hydroxy-carbonsäurederivaten der Formel

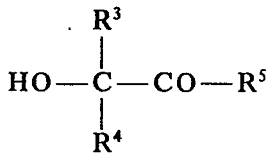

(III)

in welcher
$R^3$, $R^4$ und $R^5$ die oben angegebene Bedeutung haben,
gegebenenfalls in Gegenwart eines Säureakzeptors und gegebenenfalls in Gegenwart eines Verdünnungsmittels umsetzt.

3. Herbizide Mittel, gekennzeichnet durch einen Gehalt an mindestens einem Phenoxycarbonsäure-carbonylalkylester der Formel (I).

4. Verwendung von Phenoxycarbonsäure-carbonylalkylestern der Formel (I) zur Bekämpfung von Unkräutern.

5. Verfahren zur Herstellung von herbiziden Mitteln, dadurch gekennzeichnet, daß man Phenoxycarbonsäure-carbonylalkylester der Formel (I) mit Streckmitteln und/oder oberflächenaktiven Stoffen vermischt.

6. Verwendung von Phenoxycarbonsäure-carbonylalkylestern der Formel (I) als Defoliants und Desiccants.

**Claims**

1. Phenoxycarboxylic acid carbonylalkyl esters of the formula

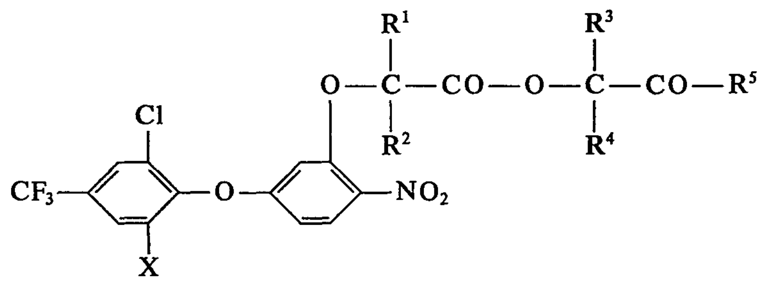

(I)

in which

$R^1$, $R^2$, $R^3$ and $R^4$ independently of one another represent hydrogen or alkyl,

$R^5$ represents hydroxy, alkoxy which is optionally substituted by alkoxy, alkylthio, dialkylamino, cyano or halogen, alkenoxy, alkinoxy, aralkoxy or aryloxy, or the radical

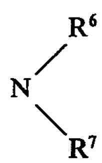

wherein

$R^6$ represents hydrogen, alkyl with 1 to 4 carbon atoms, alkenyl with 3 to 8 carbon atoms, benzyl or phenyl which is optionally substituted by alkyl with 1 to 4 carbon atoms, alkoxy with 1 to 4 carbon atoms, alkylthio with 1 to 4 carbon atoms, alkoxycarbonyl with 1 to 4 carbon atoms in the alkoxy group, fluorine, chlorine, bromine, trifluoromethyl and/or nitro, and

$R^7$ represents alkyl with 1 to 4 carbon atoms, alkenyl with 3 to 8 carbon atoms, benzyl or phenyl which is optionally substituted by alkyl with 1 to 4 carbon atoms, alkoxy with 1 to 4 carbon atoms, alkylthio with 1 to 4 carbon atoms, alkoxycarbonyl with 1 to 4 carbon atoms in the alkoxy group, fluorine, chlorine, bromine, trifluoromethyl and/or nitro, or

$R^6$ and $R^7$ together weith the nitrogen atom to which they are bonded, represent pyrrolidinyl or morpholinyl, in each case optionally substituted by 1 or 2 methyl groups, or piperidinyl, indolyl, tetrahydroindolyl, perhydroindolyl, tetrahydroquinolyl, tetrahydroisoquinolyl, perhydroquinolyl, perhydroisoquinolyl, perhydrothiazolyl or perhydroazepinyl, in each case optionally substituted by 1 to 3 methyl or ethyl groups, and

X represents hydrogen or chlorine.

2. Process for the preparation of phenoxycarboxylic acid carbonylalkyl esters of the formula

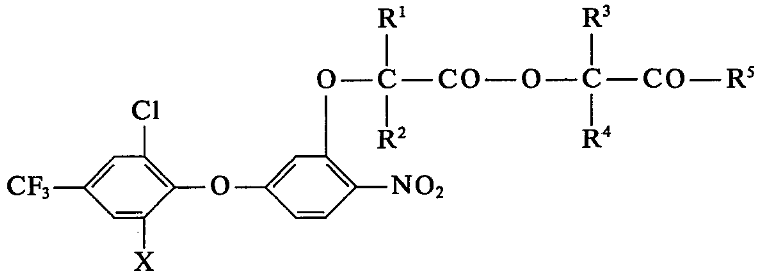

(I)

in which

$R^1$, $R^2$, $R^3$ and $R^4$ independently of one another represent hydrogen or alkyl,

$R^5$ represents hydroxy, alkoxy which is optionally substituted by alkoxy, alkylthio, dialkylamino, cyano or halogen, alkenoxy, alkinoxy, aralkoxy or aryloxy, or the radical

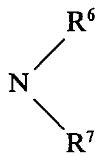

wherein

$R^6$ represents hydrogen, alkyl with 1 to 4 carbon atoms, alkenyl with 3 to 8 carbon atoms, benzyl or phenyl which is optionally substituted by alkyl with 1 to 4 carbon atoms, alkoxy with 1 to 4 carbon atoms, alkylthio with 1 to 4 carbon atoms, alkoxycarbonyl with 1 to 4 carbon atoms in the alkoxy group, fluorine, chlorine, bromine, trifluoromethyl and/or nitro, and

$R^7$ represents alkyl with 1 to 4 carbon atoms, alkenyl with 3 to 8 carbon atoms, benzyl or phenyl which is optionally substituted by alkyl with 1 to 4 carbon atoms, alkoxy with 1 to 4 carbon atoms, alkylthio with 1 to 4 carbon atoms, alkoxycarbonyl with 1 to 4 carbon atoms in the alkoxy group, fluorine, chlorine, bromine, trifluoromethyl and/or nitro, or

$R^6$ and $R^7$ together with the nitrogen atom to which they are bonded, represent pyrrolidinyl or morpholinyl, in each case optionally substituted by 1 or 2 methyl groups, or piperidinyl, indolyl, tetrahydroindolyl, perhydroindolyl, tetrahydroquinolyl, tetrahydroisoquinolyl, perhydroquinolyl, perhydroisoquinolyl, perhydrothiazolyl or perhydroazepinyl, in each case optionally substituted by 1 to 3 methyl or ethyl groups, and

X represents hydrogen or chlorine,

characterised in that phenoxycarboxylic acid chlorides of the formula

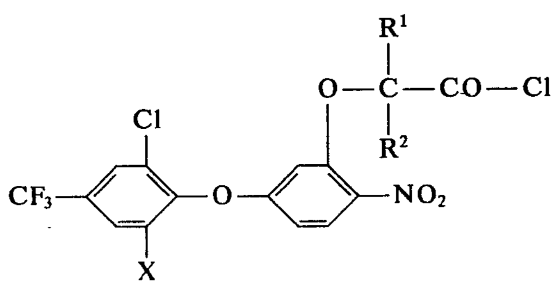

(II)

in which $R^1$, $R^2$ and X have the meaning indicated above, are reacted with $\alpha$-hydroxy-carboxylic acid derivates of the formula

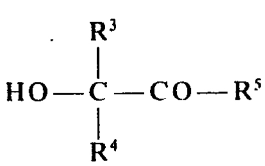

(III)

in which

$R^3$, $R^4$ and $R^5$ have the meaning indicated above, if appropriate in the presence of an acid acceptor and if appropriate in the presence of a diluent.

3. Herbicidal agents, characterised in that they contain at least one phenoxycarboxylic acid carbonylalkyl ester of the formula (I).

4. Use of phenoxycarboxylic acid carbonylalkyl esters of the formula (I) for combating weeds.

5. Process for the preparation of herbicidal agents, characterised in that phenoxycarboxylic acid carbonylalkyl esters of the formula (I) are mixed with extenders and/or surface-active substances.

6. Use of phenoxycarboxylic acid carbonylalkyl-esters of the formula (I) as defoliants and desiccants.

**Revendications**

1. Esters carbonylalcoylés d'acides phénoxycarboxyliques de formule

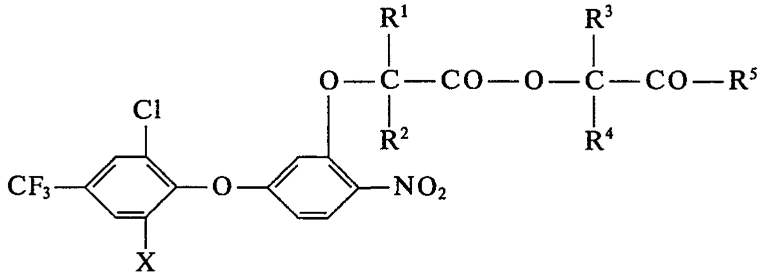

(I)

dans laquelle

$R^1$, $R^2$, $R^3$ et $R^4$ représentent indépendamment les uns des autres de l'hydrogène ou un alcoyle, hydroxy ou alcoxy éventuellement substitué par un alcoxy, alcoylthio, dialcoylamino, cyano ou de l'halogène, un alcènoxy, alcinoxy, aralcoxy ou aryloxy ou le radical

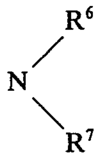

où

$R^6$ représente de l'hydrogène, un alcoyle ayant 1 à 4 atomes de carbone, alcényle ayant 3 à 8 atomes de carbone, benzyle ou phényle éventuellement substitué par un alcoyle ayant 1 à 4 atomes de carbone, alcoxy ayant 1 à 4 atomes de carbone, alcoylthio ayant 1 à 4 atomes de carbone, alcoxycarbonyle ayant 1 à 4 atomes de carbone dans le groupe alcoxy, fluor, chlore, brome, trifluorométhyle et/ou nitro, et

$R^7$ représente un alcoyle ayant 1 à 4 atomes de carbone, alcényle ayant 3 à 8 atomes de carbone, benzyle ou phényle éventuellement substitué par un alcoyle ayant 1 à 4 atomes de carbone, alcoxy ayant 1 à 4 atomes de carbone, alcoylthio ayant 1 à 4 atomes de carbone, alcoxycarbonyle ayant 1 à 4 atomes de carbone dans le groupe alcoxy, fluor, chlore, brome, trifluorométhyle et/ou nitro, ou bien

$R^6$ et $R^7$ conjointement avec l'atome d'azote auquel ils sont attachés représentent chaque fois un pyrolidinyle ou morpholinyle éventuellement substitué par 1 ou 2 groupes méthyle, pipéridinyle, indolyle, tétrahydroindolyle, perhydroindolyle, tétrahydroquinoléyle, tétrahydroisoquinoléyle, perhydroquinoléyle, perhydroisoquinoléyle, perhydrothiazolyle ou perhydroazépinyle chaque fois éventuellement substitué par 1 à 3 groupes méthyle ou éthyle, et

X représente de l'hydrogène ou du chlore.

2. Procédé de fabrication d'esters carbonylalcoylés d'acides phénoxycarboxyliques de formule

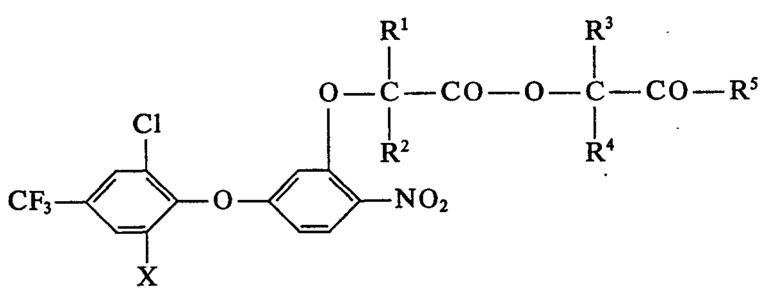

(I)

dans laquelle

$R^1$, $R^2$, $R^3$ et $R^4$ représentent indépendamment les uns des autres de l'hydrogène ou un alcoyle,

$R^5$ représente hydroxy ou alcoxy éventuellement substitué par un alcoxy, alcoylthio, dialcoylamino, cyano ou de l'halogène, un alcénoxy, alcinoxy, aralcoxy ou aryloxy ou représente le radical

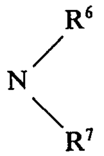

où

$R^6$ représente de l'hydrogène, alcoyle ayant 1 à 4 atomes de carbone, alcényle ayant 3 à 8 atomes de carbone, benzyle ou aryle éventuellement substitué par un alcoyle ayant 1 à 4 atomes de carbone, alcoxy ayant 1 à 4 atomes de carbone, alcoylthio ayant 1 à 4 atomes de carbone, alcoxycarbonyle ayant 1 à 4 atomes de carbone dans le groupe alcoxy, fluor, chlore, brome, trifluorométhyle et/ou nitro, et

$R^7$ représente un alcoyle ayant 1 à 4 atomes de carbone, alcényle ayant 3 à 8 atomes de carbone, benzyle ou aryle éventuellement substitué par un alcoyle ayant 1 à 4 atomes de carbone, alcoxy ayant 1 à 4 atomes de carbone, alcoylthio ayant 1 à 4 atomes de carbone, alcoxycarbonyle ayant 1 à 4 atomes de carbone dans le groupe alcoxy, fluor, chlore, brome, trifluorométhyle et/ou nitro,

ou bien

$R^6$ et $R^7$ conjointement avec l'atome d'azote auquel ils sont attachés représentent chaque fois un pyrrolidinyle ou morpholinyle éventuellement substitué par 1 ou 2 groupes méthyle, pipéridinyle, indolyle, tétrahydroindolyle, perhydroindolyle, tétrahydroquinoléyle, tétrahydroisoquinoléyle, perhydroquinoléyle, perhydroisoquinoléyle, perhydrothiazolyle ou perhydroazépinyle, chaque fois éventuellement substitué par 1 à 3 groupes méthyle ou éthyle et

X représente de l'hydrogène ou du chlore,

caractérisé en ce qu'on fait réagir des chlorures d'acides phénoxycarboxyliques de formule:

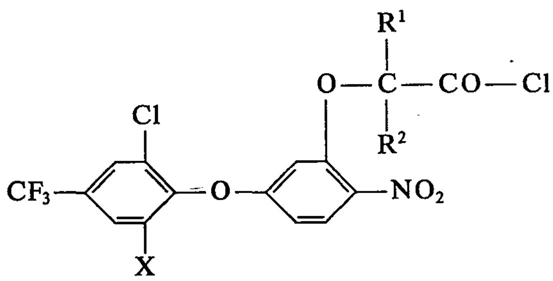

(II)

dans laquelle

$R^1$, $R^2$ et X ont la signification indiquée plus haut, avec des dérivés d'acides $\alpha$-hydroxy-carboxyliques de formule:

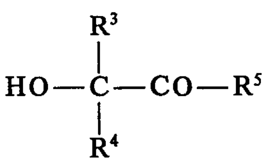

(III)

dans laquelle

$R^3$, $R^4$ et $R^5$ ont la signification indiquée plus haut,

éventuellement en présence d'un accepteur d'acide et éventuellement en présence d'un diluant.

3. Agents herbicides, caractérisés par une teneur en au moins un ester carbonylalcoylé d'acide phénoxycarboxylique de formule (I).

4. Utilisation des esters carbonylalcoylés d'acides phénoxycarboxyliques de formule (I) pour combattre les plantes adventices.

5. Procédé de préparation d'agents herbicides, caractérisé en ce qu'on mélange des esters carbonylalcoylés d'acides phénoxycarboxyliques de formule (I) avec des diluants et/ou des substances tensioactives.

6. Utilisation d'esters carbonylalcoylés d'acides phénoxycarboxyliques de formule (I) comme défoliants et dessiccants.